# NOUVEAU FASCICULE DE BREVET EUROPEEN

(11) **EP 0 720 845 B2**
(45) Date de publication et mention de la décision concernant l'opposition: **27.08.2003**
(45) Mention de la délivrance du brevet: 09.07.1997
(21) Numéro de dépôt: 95119625.2
(22) Date de dépôt: 13.12.1995
(51) Int. Cl.: A61K 7/48, A61K 7/02, A61K 7/025

(54) **Composition cosmétique sous forme de pâte souple et procédé de préparation**
Kosmetische Zusammensetzung in Form einer weichen Paste und ihr herstellungsverfahren
Cosmetic composition in form of a supple paste and preparation process

(30) Priorité: 05.01.1995 FR 9500072
(43) Date de publication de la demande: 10.07.1996
(73) Titulaire: L'OREAL, 75008 Paris (FR)
(72) Inventeur: Le Bras-Roulier, Veronique, F-75002 Paris (FR); Miguel-Colombel, Dolores, F-92340 Bourg La Reine (FR); Pradier, Francoise, F-92260 Fontenay aux Roses (FR)
(74) Mandataire: Dossmann, Gérard

(56) Documents cités:
- EP-A- 0 524 892
- EP-A- 0 530 084
- EP-A- 0 605 284
- EP-A- 0 609 132
- EP-A- 0 667 146
- WO-A-91/16879
- DE-A- 3 243 017
- DE-A- 3 744 352
- FR-A- 2 486 800
- GB-A- 2 167 301
- GB-A- 2 216 797

## Description

La présente invention a trait à une composition se présentant sous forme d'une pâte souple, notamment une composition cosmétique pouvant être utilisée pour le maquillage des lèvres, ou comme composition pour le traitement de la peau et/ou des lèvres.

Les compositions cosmétiques pouvant être appliquées sur les lèvres comme produit de soin ou de maquillage, tels les rouges à lèvres, contiennent généralement des corps gras et des cires, et eventuellement des additifs et des pigments.

On connaît en particulier de telles compositions se présentant sous forme d'une pâte souple, susceptibles d'être appliquées après prélèvement à l'aide d'un applicateur, par example un pinceau.
Ces compositions pouvent contenir des cires ce qui leur confère des qualités de consistance, d'onctuosité et de tenue du film applique remarquables.
On connaît, par EP657145, une composition cosmétique sous forme de pâte souple, comprenant dans une phase grasse, une cire ayant un point de fusion supérieur à 55°C, ladite composition ayant une viscosité dynamique de 3-30 Pa.s.
On connaît également EP805284 qui décrit une composition solide ou pâteuse comprenant une phase grasse et une phase pulvérulante partiellement constituée d'une poudre légère.
On connaît encore par FR2486800, une crème cosmétique anhydre et onctueuse, comprenant une cire, une huile et une montmorillonite et/ou de la vaseline et/ou une graisse de polyéthylène, ladite crème ayant une viscosité de 10-100 poises.
On connaît par GB2216797, un mascara comprenant une cire de point de fusion de 80-100°C et un hydrolysat de kératine de poids moléculaire compris entre 50000 et 200000.
On connaît par EP580084, une composition pour le maquillage ou le soin des cils ou sourcils, comprenant une dispersion dans laquelle la phase dispersée comporte au moins une cire hydrocarbonée et une charge, et la phase continue est une phase aqueuse comprenant un polymère hydrosoluble.
On connaît par WO91/16879, une composition comprenant une cire, un triglycéride, un mélange d'esters, un phosphate, un agent gonflant et un colorant.

Afin d'introduire des quantités relativement importantes de cires dans ces compositions. Il a été proposé un procédé de préparation consistant à préparer le mélange des constituants, à le chauffer jusqu'à une température à laquelle les cires sont au moins partiellement fondues, puis à le soumettre à une opération de malaxage pendant au moins une partie de son refroidissement.
On observe ainsi une cristallisation des cires sous une forme permettant l'obtention d'une pâte souple et facilement prélevable.
On a toutetois constaté que certaines cires ne permettaient pas d'obtenir une composition cosmétique présentant des qualités cosmétiques optimales et constantes au cours de leur conservation, en particulier lorsque cette conservation était effectuée, même partiellement, à une température supérieure à une température usuelle de l'ordre de 20-25°C. En effet, selon la nature des cires présentes dans la composition, on a pu constater dans certains cas, une modification de la viscosité de ladite composition, pouvant entraîner un durcissement important de la composition et des difficultés d'application.

La présente invention a pour but de pallier ces inconvénients et de proposer une composition cosmétique présentant des qualités cosmétiques optimales tout au long de sa conservation, entre autre une viscosité quasi-constante au cours du temps, quelle que soit la température ou les changements de température subis.

Un objet de la présente invention est donc une compositon cosmétique se présentant sous forme d'une pâte souple et comprenant une phase grasse dans laquelle sont présentes une ou plusieurs cires, ladite composition étant caractérisée par le fait qu'au moins 85% desdites cires ont une température de fusion commençante supérieure ou égale à 50°C.

Un autre objet de l'invention est un procédé de préparation d'une telle composition cosmétique, dans lequel on porte à une température à laquelle les cires, fondent au moins partiellement, au moins une partie des différents constituants de la composition dont les cires on ajoute le cas échéant le reste des constituants, puis l'on malaxe le mélange obtenu pendant son refroidissement.

La présente invention permet l'obtention de compositions ou restent stables dans le temps, c'est-à-dire dont la viscosité reste quasi-constante. Ces compositions possèdent une texture souple originale et présentent, après application, une bonne tenue et une bonne brillance.

La composition selon l'invention comprend donc une phase grasse dans laquelle sont présentes une ou plusieurs cires caractérisée par le fait
- qu'elle se présente sous forme d'une pâte souple ayant une viscosité dynamique à 25°C de 3 à 35 Pa.s,
- qu'au moins 95% desdites cires ont une température de fusion commençante supérieure ou égale à 50°C,
- que la composition est susceptible d'être obtenue par mélange d'au moins une partie des constituants de la composition dont au moins les cires, chauffage à une température à laquelle lesdites cires fondent au moins partiellement, ajout le cas échéant du reste des constituants, puis malaxage du mélange obtenu pendant son refroidissement jusqu'à température ambiante.

Par température de fusion commençante, on entend dans la présente description la température à laquelle une cire commence à fondre.

On peut déterminer cette température par ATD (analyse thermique différentielle) qui permet l'obtention du thermogramme (ou courbe de fusion) de la cire considérée. La température de fusion commençante correspond à la température à laquelle on peut observer un changement de pente notable dans le thermogramme. Le point de fusion, quant à lui, représente le point minimum dudit thermogramme.

Sans être tenu par la présente explication, on peut supposer que le changement de viscosité observé lors de la conservation de certaines compositions cosmétiques est lié à la modification de la forme dans laquelle les cires cristallisent.
En effet, lors de la fabrication de la composition, les cires sont cristallisées dans une certaine forme qui va permettre l'obtention d'une pâte souple.
Lors d'une élévation importante de température au cours de leur conservation, lesdites cires peuvent fondre au moins partiellement, puis recristalliser dans une forme différente de la forme cristalline initiale, cette seconde forme ne permettant plus d'obtenir une pâte souple mais pouvant conduire à une pâte de viscosité plus importante et donc de consistance plus rigide.
Ainsi, en choisissant au moins 95% des cires présentes dans la composition parmi les cires ayant une température de fusion commençante supérieure ou égale à 50°C, on peut obtenir une composition dont la viscosité reste constante.
De préférence, on utilise des cires dont la température de fusion commençante est supérieur à 65°C et/ou l'on utilise 100% de cires dont la température de fusion commençante est supérieure ou égale à 50°C.

Les cires susceptibles d'être utilisées dans la présente invention, c'est-à-dire permettant de conserver une viscosité de la composition cosmétique constante, peuvent être de toute nature, en particulier d'origine minérale, animale, végétale ou synthétique.

On peut principalement citer la cire de Carnauba, certaines cires de polyéthylène et certaines cires microcristallines telles que celle vendue par Tisco sous le nom « Tisco Wax 88 ».
Ces cires peuvent être utilisées seules ou en mélange.
Elles peuvent également être utilisées en mélange avec des cires dont la température de fusion commençante est inférieure à 50°C, étant donné que ces secondes cires ne peuvent pas représenter plus de 5% en poids de la totalité des cires.

La composition selon l'invention peut comprendre au total 10-60% en poids de cires par rapport au poids final de la composition, de préférence 15-35%.

La phase grasse peut en outre comprendre, d'autres constituants gras tels que des huiles. On peut en particulier citer :
. les huiles minérales telles que l'huile de paraffine ou de vaseline,
. les huiles animales telles que le perhydrosqualène ou l'huile d'arara,
. les huiles végétales telles que l'huile d'amande douce, de calophyllum, de palme, de ricin, d'avocat, de jojoba, doive ou de germes de céréales,
. des esters d'acide lanolique, d'acide oléique, d'acide laurique, d'acide stéarique ou d'acide myristique par exemple,
. des alcools tels que l'alcool oléique, l'alcool linoléique ou linolénique, l'alcool isostéarique ou l'octyl dodécanol,
. des acétylglycérides, des octanoates, décanoates ou ricinoléates d'alcools ou de polyalcools.
Ces constituants gras peuvent représenter 40-90% en poids de la composition, de préférence 65-85%.

De façon connue, on peut ajouter à la composition selon l'invention un agent de coloration pulvérulent tel que le noir de carbone, les oxydes de chrome ou de fer, les outremers, le pyrophosphate de manganèse, le bleu ferrique, le dioxyde de titane, les agents nacrants généralement utilisés en mélange avec des pigments colorés ou certains colorants organiques généralement utilisés en mélange avec des pigments colorés et couramment utilisés dans l'industrie cosmétique.
Ces agents de coloration peuvent être présents en une quantité de 0 à 20%.

On peut également ajouter des charges pulvérulentes minérales ou organiques, généralement en une quantité de 0 à 40%.
Ces charges pulvérulentes peuvent être choisies parmi le talc, les micas, le kaolin, les oxydes de zinc ou de titane, les carbonates de calcium ou de magnésium, la silice, le dioxyde de titane sphérique, les billes de verre et de céramique, les savons métalliques dérivés d'acides carboxyliques ayant 8-22 atomes de carbone, les poudres de polymères synthétiques non expansées, les poudres expansées et les poudres de composés organiques naturels tels que les amidons de céréales, réticulés ou non.

On peut encore ajouter tout additif usuellement utilisé dans l'industrie cosmétique, tel que des antioxydants, des parfums, des conservateurs, ainsi que des actifs cosmétiques et/ou pharmaceutiques tels que des dérivés de vitamines, des acides gras essentiels, des sphingocérils, des filtres solaires liposolubles, des anti-inflammatoires ou des extraits huileux de plantes, voire des polymères liposolubles, et/ou des huiles et/ou gommes de silicone telles que les diméthylpolysiloxanes. Ces additifs peuvent être présents à raison de 0-10% en poids.

Afin de préparer la composition selon l'invention, on peut tout d'abord préparer un prémélange comprenant au moins une partie des différents constituants de la composition, dont au moins la cire selon l'invention, chauffer ce prémélange à une température à laquelle ladite cire fond au moins partiellement, ajouter le cas échéant le reste des constituants, puis malaxer le mélange obtenu pendant au moins une partie de son refroidissement jusqu'à température ambiante.
On a en effet constaté que lorsque l'on malaxe le mélange lors d'au moins une partie de son refroidissement, de manière à créer des zones de cisaillement, on obtient une composition se présentant sous forme de pâte homogène et souple.
II semblerait, sans toutefois être tenu par cette explication, que dans ces conditions la cire cristallise sous forme de fins cristaux, ce qui expliquerait que la composition reste sous forme de pâte souple.

On peut effectuer l'opération de chauffage selon toute technique connue.
L'opération de malaxage peut être effectuée, par exemple, dans un broyeur à cylindres comportant deux cylindres tournant en sens inverse entre lesquels passe la pâte, ou encore dans un mélangeur-extrudeur qui permet d'obtenir une pâte de qualité très constante de façon reproductible. De plus, il est possible, en adaptant la filière de sortie du mélangeur-extrudeur, de conditionner la composition en ligne à la sortie de ladite filière.
Dans un mode particulier de réalisation de l'invention, les opérations de mélange, de chauffage et/ou de malaxage/cisaillement, voire de refroidissement, sont réalisées dans un ou plusieurs extrudeurs disposés à la suite les uns des autres, de préférence dans un extrudeur bivis unique.
En effet, la composition obtenue après extrusion présente une douceur particulière, et procure une certaine sensation de glissant lorsqu'elle est appliquée sur la peau, tout en évitant l'aspect et la sensation de gras huileux.
Les conditions dans laquelle l'extrusion peut être effectuée sont décrites dans la demande de brevet FR94-00756.

On obtient ainsi une composition à usage topique, qui peut être appliquée sur la peau et/ou sur les lèvres en tant que produit de maquillage, rouge à lèvres par exemple, ou en tant que produit de soin.

La composition se présente sous forme de pâte souple, dont on peut mesurer la viscosité, par opposition à la structure solide d'un bâton, ou stick, dont on ne peut pas mesurer la viscosité. Ladite viscosité dynamique à 25°C est généralement comprise entre 3 et 35 Pa.s, mesurée avec un viscosimètre rotatif CONTRAVES TV équipé d'un mobile "MS-r4" à la fréquence de 60 Hz.

L'invention est illustrée plus en détail dans les exemples suivants, dans lesquels les pourcentages sont donnés en poids.
Les mesures de viscosité sont effectuées à 25°C, à l'aide d'un viscosimètre rotatif Contraves équipé d'un mobile MS-R4.
Les mesures des caractéristiques de fusion des cires est faite par ATD (analyse thermique différentielle) dans les conditions suivantes : chauffage de 25 à 110°C, à une vitesse de 1°C/minute.

### Exemple 1 : Composition selon l'invention

On prépare un rouge à lèvres ayant la composition suivante:

| | |
|---|---|
| . huile de vaseline | 22,5% |
| . huile de lanoline | 23,5% |
| . lanolate d'isopropyle | 24% |
| . cire microcristalline (cire minérale non polaire vendue par Tisco, sous le nom « Tisco wax 88 ») | 20% |
| . charges (oxyde de titane, mica) | 3,5% |
| . pigments | 6,5% |

On mélange ces différents ingrédients à environ 100°C et l'on introduit le mélange en tête d'un extrudeur bi-vis. L'extrusion est effectuée dans les conditions suivantes:
. température d'entrée : 100°C
. température de sortie : 30°C
. temps de résidence : 3 minutes environ
. vitesse des vis : 350 tours/min

On obtient en sortie une pâte souple, se présentant sous forme d'une seule phase stable et homogène, et pouvant être prélevée à l'aide d'un pinceau pour son application.

Après application, cette pâte est considérée comme présentant des qualités de douceur et de glissant satisfaisantes et ne présente pas de texture huileuse.

Après six mois de conservation, à 25°C et 47°C, on obtient les mesures de viscosité suivantes:
. conservation à 25°C : 90 poises (9 Pa.s)
. conservation à 47°C : 92 poises (9,2 Pa.s)
On constate donc que la composition selon l'invention conserve quasiment la même viscosité après conservation à température ambiante ou à température élevée.
Après six mois de conservation à 47°C, la pâte n'a pas changé d'aspect et peut toujours être prélevée à l'aide d'un pinceau pour être appliquée sur la peau.
La cire utilisée dans cette composition possède une température de fusion commençante de l'ordre de 65-70°C et un point de fusion à 91-93°C environ.

### Exemple 2 : Exemple comparatif

On prépare un rouge à lèvres ayant la composition suivante:

| | |
|---|---|
| . huile de vaseline | 22,5% |
| . huile de lanoline | 23,5% |
| . lanolate d'isopropyle | 24% |
| . cire microcristalline (cire minérale non polaire vendue par RMC, sous le nom de « Feruwax 30540 ») | 20% |
| . charges (oxyde de titane, mica) | 3,5% |
| . pigments | 6,5% |

On prépare le rouge à lèvres de la même manière que dans l'exemple 1, et l'on obtient également une pâte souple susceptible d'être prélevée à l'aide d'un pinceau.

Après conservation pendant deux mois, on obtient les mesures de viscosité suivantes :
. conservation à 25°C : 80 poises (8 Pa.s)
. conservation à 47°C : 175 poises (17,5 Pa.s)
On constate donc qu'après deux mois seulement de conservation à 47°C, on obtient une composition très épaissie, qui ne peut plus sortir de l'applicateur.
La cire utilisée, bien qu'ayant un point de fusion à environ 69-71°C, a une température de fusion commençante de l'ordre de 45-46°C.
Ainsi, le simple fait de choisir une cire dont le point de fusion est de l'ordre de la température de conservation n'est pas suffisant en soi pour permettre de garder une viscosité constante à la composition et donc de ses propriétés cosmétiques convenables.

## Revendications

1. Composition cosmétique comprenant une phase grasse dans laquelle sont présentes une ou plusieurs cires **caractérisée par le fait**
- **qu'**elle se présente sous forme d'une pâte souple ayant une viscosité dynamique à 25°C de 3 à 35 Pa.s,
- **qu'**au moins 95% desdites cires ont une température de fusion commençante supérieure ou égale à 50°C,
- **que** la composition est susceptible d'être obtenue par mélange d'au moins une partie des constituants de la composition dont au moins les cires, chauffage à une température à laquelle lesdites cires fondent au moins partiellement, ajout le cas échéant du reste des constituants, puis malaxage du mélange obtenu pendant son refroidissement jusqu'à température ambiante.

2. Composition selon la revendication 1, dans laquelle 100% des cires ont une température de fusion commençante supérieure à 50°C.

3. Composition selon l'une des revendications précédentes, dans laquelle les cires ont une température de fusion commençante supérieure à 65°C.

4. Composition selon l'une des revendications précédentes, dans laquelle les cires sont choisies parmi la cire de Carnauba, certaines cires de polyéthylène et certaines cires microcristallines.

5. Composition selon l'une des revendications précédentes, dans laquelle les cires représentant 10-60%, de préférence 15-35%, en poids de la composition.

6. Composition selon l'une des revendications précédentes, dans laquelle la phase grasse comprend en outre d'autres constituants gras choisis pamri les huiles.

7. Composition selon la revendication 6, dans laquelle les autres constituants gras sont choisis parmi l'huile de paraffine ou de vaseline, le perhydrosqualène ou l'huile d'arara, l'huile d'amande douce, de calophylium, de palme, de ricin, d'avocat, de jojoba, d'olive ou de germes de céréales, des esters d'acide lanolique, d'acide oléique, d'acide laurique, d'acide stéarique ou d'acide myristique, des alcools tels que l'alcool oléique, l'alcool linoléique ou linolénique, l'alcool isostéarique ou l'octyl dodécanol, des acétylglycérides, des octanoates, décanoates ou ricinoléates d'alcools ou de polyalcools.

8. Composition selon l'une des revendicalions 5 à 7, dans laquelle les autres constituants gras représentent 40-90%, de préférence 65-85%, en poids de la composition.

9. Composition selon l'une des revendications précédentes, comprenant en outre un agent de coloration pulvérulent tel que le noir de carbone, les oxydes de chrome ou de fer, les outremers, le pyrophosphate de manganése, le bleu ferrique, le dioxyde de titane, les agents nacrants et certains colorants organiques.

10. Composition selon l'une des revendications précédentes, comprenant en outre des charges pulvérulentes minérales ou organiques, telles que le talc, les micas, le kaolin, les oxydes de zinc ou de titane, les carbonates de calcium ou de magnésium, la silice, le dioxyde de titane sphérique, les billes de verre et de céramique, les savons métalliques dérivés d'acides carboxyliques ayant 8-22 atomes de carbone, les poudres de polymères synthétiques non expansées, les poudres expansées et les poudres de composés organiques naturels tels que les amidons de céréales, réticulés ou non.

11. Composition selon l'une des revendications précédentes, se présentant sous la forme d'un produit de maquillage et/ou de soin des lèvres.

12. Procédé de préparation d'une composition se présentant sous forme d'une pâte souple ayant une viscosité dynamique à 25°C de 3 à 35 Pa.s et comprenant une phase grasse dans laquelle sont présentes une ou plusieurs cires, 95% desdites cires ayant une température de fusion commençante supérieure ou égale à 50°C, dans lequel on porte à une température à laquelle les cires fondent au moins partiellement, au moins une partie des différents constituants de la composition dont les cires, on ajoute le cas échéant le reste des constituants, puis l'on malaxe le mélange obtenu pendant son refroidissement jusqu'à température ambiante.

13. Procédé selon la revendication 12, dans lequel l'opération de malaxage est effectuée dans un broyeur à cylindres ou dans un extrudeur.

14. Procédé selon l'une quelconque des revendications 12 à 13, dans lequel les opérations de mélange, chauffage et/ou malaxage sont réalisées dans un ou plusieurs extrudeurs disposés à la suite les uns des autres.

15. Procédé selon l'une des revendications 12 a 14, dans lequel les opérations de mélange, chauffage et malaxage sont réalisées dans un extrudeur bivis unique.

## Patentansprüche

1. Kosmetische Zusammensetzung, die eine Fettphase enthält, in der ein oder mehrere Wachse vorliegen, **dadurch gekennzeichnet, daß**
- sie in Form einer weichen Paste vorliegt, die bei 25 °C eine dynamische Viskosität von 3 bis 35 Pa·s aufweist,
- mindestens 95 % der Wachse eine beginnende Schmelztemperatur von 50 °C oder darüber aufweisen, und
- die Zusammensetzung erhältlich ist, indem zumindest ein Teil der verschiedenen Bestandteile der Zusammensetzung und darunter zumindest die Wachse auf eine Temperatur erwärmt werden, bei der die Wachse zumindest teilweise schmelzen, ggf. die übrigen Bestandteile zugegeben werden und dann das so hergestellte Gemisch beim Abkühlen auf Raumtemperatur geknetet wird.

2. Zusammensetzung nach Anspruch 1, wobei 100 % der Wachse eine beginnende Schmelztemperatur über 50 °C aufweisen.

3. Zusammensetzung nach einem der vorhergehenden Ansprüche, wobei die Wachse eine beginnende Schmelztemperatur über 65 °C aufweisen.

4. Zusammensetzung nach einem der vorhergehenden Ansprüche, wobei die Wachse unter Carnaubawachs, verschiedenen Polyethylenwachsen und verschiedenen mikrokristallinen Wachsen ausgewählt sind.

5. Zusammensetzung nach einem der vorhergehenden Ansprüche, wobei die Wachse 10 bis 60 % und vorzugsweise 15 bis 35 % des Gewichts der Zusammensetzung ausmachen.

6. Zusammensetzung nach einem der vorhergehenden Ansprüche, wobei die Fettphase ferner weitere Fettbestandteile enthält, die unter den Ölen ausgewählt sind.

7. Zusammensetzung nach Anspruch 6, wobei die weiteren Fettbestandteile unter Paraffinöl oder Vaselineöl, Perhydrosqualen oder Araraöl, süßem Mandelöl, Calophyllumöl, Palmöl, Ricinusöl, Avocadoöl, Jojobaöl, Olivenöl oder Öl von Getreidekeimen, Estern von Lanolinsäure, Ölsäure, Laurinsäure, Stearinsäure oder Myristinsäure, Alkoholen, wie Oleylalkohol, Linoleyl- oder Linolenylalkohol, Isostearylalkohol der Octyldodecanol, Acetylglyceriden, Octanoaten, Decanoaten oder Ricinoleaten von Alkoholen oder Polyalkoholen ausgewählt sind,

8. Zusammensetzung nach einem der Ansprüche 6 bis 7, wobei die weiteren Fettbestandteile 40 bis 90 Gew.-% und vorzugsweise 65 bis 85 Gew.-% der Zusammensetzung ausmachen.

9. Zusammensetzung nach einem der vorhergehenden Ansprüche, die ferner ein pulverförmiges Färbemittel aufweist, wie Ruß, Chrom- oder Eisenoxid, Ultramarine, Manganpyrophosphat, Eisenblau, Titandioxid, Glanzmittel und verschiedene organische Färbemittel.

10. Zusammensetzung nach einem der vorhergehenden Ansprüche, die ferner anorganische oder organische pulverförmige Füllstoffe enthält, wie Talk, Glimmer, Kaolin, Zink- oder Titanoxid, Calcium- oder Magnesiumcarbonat, Siliciumdioxid, sphärisches Titandioxid, Glas- und Keramikkugeln, Metallseifen, die von Carbonsäuren mit 8 bis 22 Kohlenstoffatomen abgeleitet sind, synthetische nicht expandierte Polymerpulver, expandierte Pulver und Pulver von natürlichen organischen Verbindungen, wie Stärke von Cerealien, die ggf. vernetzt sind.

11. Zusammensetzung nach einem der vorhergehenden Ansprüche, die in Form eines Produkts zum Schminken und/oder zur Pflege der Lippen vorliegt.

12. Verfahren zur Herstellung einer Zusammensetzung, die in Form einer weichen Paste vorliegt, welche bei 25 °C eine dynamische Viskosität von 3 bis 35 Pa·s aufweist, und die eine Fettphase enthält, in der ein oder mehrere Wachse vorliegen, wobei 95 % der Wachse eine beginnende Schmelztemperatur von 50°C oder darüber aufweisen, wobei nach dem Verfahren mindestens ein Teil der verschiedenen Bestandteile der Zusammensetzung und darunter die Wachse auf eine Temperatur erwärmt werden, bei der die Wachse zumindest teilweise schmelzen, ggf. die restlichen Bestandteile zugegeben werden und anschließend das hergestellte Gemisch beim Abkühlen auf Raumtemperatur geknetet wird.

13. Verfahren nach Anspruch 12, wobei der Arbeitsgang des Knetens in einer Walzenmühle oder einem Extruder durchgeführt wird.

14. Verfahren nach einem der Ansprüche 12 bis 13, wobei die Arbeitsgänge des Mischens, Erwärmens und/oder Knetens in einem oder mehreren Extrudern durchgeführt werden, die nacheinander angeordnet sind.

15. Verfahren nach einem der Ansprüche 12 bis 14, wobei die Arbeitsgänge des Mischens, Erwärmens und/oder Knetens in einem einzigen Zweischneckenextruder durchgeführt werden.

## Claims

1. Cosmetic composition comprising a fatty phase in which one or more waxes are present, **characterized in that**
- it is presented in the form of a soft paste having a dynamic viscosity at 25°C of 3 to 35 Pa.s,
- at least 95 % of the said waxes have a temperature at onset of melting greater than or equal to 50°C,
- the composition can be obtained by mixing at least a portion of the constituents of the composition, including at least the waxes, heating to a temperature at which the said waxes melt at least partially, adding the remainder of the constituents where appropriate, and then blending the mixture obtained while it cools to room temperature.

2. Composition according to Claim 1, in which 100 % of the waxes have a temperature at onset of melting greater than 50°C.

3. Composition according to one of the preceding claims, in which the waxes have a temperature at onset of melting greater than 65°C.

4. Composition according to one of the preceding claims, in which the waxes are chosen from Carnauba wax, some polyethylene waxes and some microcrystalline waxes.

5. Composition according to one of the preceding claims, in which the waxes represent 10-60 %, preferably 15-35 %, by weight of the composition.

6. Composition according to one of the preceding claims, in which the fatty phase comprises, in addition, other fatty constituents chosen from oils.

7. Composition according to Claim 6, in which the other fatty constituents are chosen from paraffin oil or liquid paraffin, perhydrosqualene or arara oil, sweet almond, calophyllum, palm, castor, avocado, jojoba, olive or cereal germ oil, esters of lanolic acid, oleic acid, lauric acid, stearic acid or myristic acid, alcohols such as oleyl alcohol, linoleyl or linolenyl alcohol, isostearyl alcohol or octyl dodecanol, acetylglycerides, octanoates, decanoates or ricinoleates of alcohols or of polyalcohols.

8. Composition according to either of Claims 6 and 7, in which the other fatty constituents represent 40-90 %, preferably 65-85 %, by weight of the composition.

9. Composition according to one of the preceding claims, comprising, in addition, a pulverulent colouring agent such as carbon black, chromium or iron oxides, ultramarines, manganese pyrophosphate, ferric blue, titanium dioxide, pearlescent agents and certain organic colorants.

10. Composition according to any one of the preceding claims, comprising, in addition, inorganic or organic pulverulent fillers, such as talc, micas, kaolin, zinc or titanium oxides, calcium or magnesium carbonates, silica, spheric titanium dioxide, glass and ceramic beads, metallic soaps derived from carboxylic acids having 8-22 carbon atoms, nonexpanded synthetic polymer powders, expanded powders and powders of natural organic compounds such as cereal starches, crosslinked or otherwise.

11. Composition according to one of the preceding claims, which is presented in the form of a lip make-up and/or treatment product.

12. Process for the preparation of a composition which is presented in the form of a soft paste having a dynamic viscosity at 25°C of 3 to 35 Pa.s and which comprises a fatty phase in which one or more waxes are present, 95 % of the said waxes having a temperature at onset of melting greater than or equal to 50°C, in which at least a portion of the various constituents of the composition, including the waxes, is heated to a temperature at which the waxes melt at least partially, the remainder of the constituents are added where appropriate, and then the mixture obtained is blended while it cools to room temperature.

13. Process according to Claim 12, in which the blending operation is carried out in a roll mill or in an extruder.

14. Process according to either of Claims 12 and 13, in which the mixing, heating and/or blending operations are carried out in one or more extruders arranged successively one after the other.

15. Process according to one of Claims 12 to 14, in which the mixing, heating and blending operations are carried out in a single twin-screw extruder.
